# EUROPEAN PATENT APPLICATION

(11) **EP 2 065 048 A1**
(43) Date of publication of application: **03.06.2009**
(21) Application number: 07291431.0
(22) Date of filing: 30.11.2007
(51) Int. Cl.: A61K 35/74, A61P 37/00, G01N 33/50, G01N 33/573, G01N 33/68, A23L 1/30

(54) **Use of a L. casei strain, for the preparation of a composition for inhibiting mast cell activation**

(71) Applicant: INSTITUT PASTEUR, 75015 Paris (FR); Compagnie Gervais Danone, 75009 Paris (FR)
(72) Inventor: Schiffer-Mannioui, Cécile, 75008 Paris (FR); Daëron, Marc, 78460 Chevreuse (FR); Samson, Sandrine, 69003 Lyon (FR); Bourdet-Sicard, Raphaëlle, 91120 Palaiseau (FR)
(74) Representative: Marcadé, Véronique

(57) **Abstract**

The present invention pertains to the field of prevention and treatment of chronic or acute diseases involving mast cells, such as allergy and some autoimmune diseases. More precisely, the present invention concerns the use of probiotics containing a *L*. *casei* strain and/or a *B. breve* strain for inhibiting mast cell activation. The present invention relates also to screening processes for identifying bacterial strains which can be used for preparing said probiotics.

## Description

The present invention pertains to the field of prevention and treatment of chronic or acute diseases involving mast cells, such as allergy and some autoimmune diseases. More precisely, the present invention concerns the use of probiotics for inhibiting mast cell activation.

Mast cells are well known as major players in allergies. Allergic reactions depend primarily on IgE antibodies. Mast cells express high-affinity IgE receptors (FcεRI), a proportion of which are occupied by IgE antibodies *in vivo*. When aggregated at the cell surface upon binding of a multivalent allergen to IgE antibodies, FcεRI transduce activation signals which lead to mast cell activation. Activated mast cells release and secrete a variety of inflammatory molecules. These include preformed vasoactive amines and enzymes stored in mast cell granules (Miller and Pemberton, 2002), newly formed lipid-derived prostaglandins, thromboxanes and leukotrienes (Triggiani et al., 1995), newly transcribed cytokines (Galli et al., 1991), growth factors and chemokines (Kaplan, 2001). These mediators have a wide array of biological effects. They increase vascular permeability, trigger the contraction of smooth muscles, attract and activate numerous inflammatory cells. Altogether, they concur to generate an acute reaction within minutes, followed by a late reaction within hours, a chronic reaction within days, and tissue remodelling within months.

Mast cells express not only IgE receptors, but also IgG receptors (FcγR) (Benhamou et al., 1990; Daeron et al., 1980). These include activating receptors (FcγRIIIA in mice, FcγRIIA in humans) and inhibitory receptors (FcγRIIB in both species). When aggregated by IgG immune complexes, activating FcγR trigger the same biological responses as FcεRI (Daeron et al., 1992; Hazenbos et al., 1996; Latour et al., 1992). When co-aggregated with FcεRI or with activating FcγR by immune complexes, FcγRIIB negatively regulate IgE- or IgG-induced mast cell activation (Daeron et al., 1995a; Daeron et al., 1995b). It was recently found, in murine models of autoimmune arthritis (Lee et al., 2002) and encephalitis (Robbie-Ryan et al., 2003), that mast cells play critical roles in IgG-dependent tissue-specific autoimmune inflammation. The clinical expression of these pathological conditions was indeed abrogated in mast cell-deficient mice. Moreover, the pathogenesis of these diseases critically depended on the ratio between activating and inhibitory FcγRs on mast cells, and on their engagement by IgG antibodies (Nigrovic et al., 2007; Robbie-Ryan et al., 2003).

Besides their roles in inflammation, mast cells were recently understood to interact with microorganisms and to contribute to the protection against pathogens. They are present in virtually all tissues, particularly in those which are at the interface with the external milieu (skin, tongue, stomach, gut, lungs...). They express Toll-like Receptors (TLR) and other receptors which enable their interactions with bacteria and with soluble molecules of microbial origin, including endotoxins, CpG nucleotides, peptidoglycans and lipopeptides. When engaged by their ligands, most of these receptors can transduce signals which also lead to the secretion of pro-inflammatory mediators (Arock et al., 1998). Besides, mast cells can phagocytose bacteria (Arock et al., 1998). The protective role of mast cells in bacterial infections was dramatically demonstrated in the murine model of peritonitis induced by cecal ligation and puncture: whereas most wild-type mice survive the severe peritonitis which develops following this aggression, most mast cell-deficient mice die (Shelley et al., 2003; Supajatura et al., 2001).

On the basis of the above data, mast cells now appear as cells of the innate immune system which, because they express FcRs, can be enrolled in adaptive immunity by antibodies. They are thus well suited for innate and adaptive immunity to meet and interfere with each other. The inventors hence investigated the possible cross-talk between innate and adaptive immunity in mast cells. Specifically, they chose to investigate whether and how probiotics may interfere with IgE- and IgG-induced mast cell activation. As exemplified in the experimental part below, they demonstrated that some probiotics are able to inhibit mast cell activation, thereby having protective effects against certain human inflammatory diseases, including autoimmune diseases and allergies.

A first aspect of the present invention is hence the use of a *L*. *casei* strain and/or a *Bifidobacterium breve* strain, for the preparation of a composition for inhibiting mast cell activation.

Depending on the clinical context and on the route of administration, the compositions prepared according to the invention can inhibit IgE- and/or IgG-induced mast cell activation. They can hence be used to prevent, alleviate and/or treat any inflammatory manifestation implying mast cell activation by antibodies in the presence of antigens. In particular, when the IgE-induced activation is inhibited, these compositions can be used for preventing, alleviating and/or treating an allergy. The allergies considered herein are caused by IgE antibodies which bind to mast cells and, when recognizing specific antigens, trigger their activation. A huge number of antigens can cause allergies, which can manifest themselves in a great variety of clinical symptoms. Non-limitative examples of antigens frequently at the origin of allergies are environmental allergens such as mite (*e*.*g*., Der p 2), cockroach antigens, birch pollen (*e*.*g.* Bet V 1), grass pollen, animal hair dander antigens (*e*.*g*., Cat: Fel d 1), bee venom (*e*.*g*., phospholipase), or food allergens such as milks (especially cow milk), peanut, shrimp, soya, eggs, cereal products, fruits, etc. Depending on the context and the individual, the clinical symptoms can be local (which is the case, for example, in allergic rhinitis, conjunctivitis or otitis), regional (*e.g*., asthma, dermatitis, gastroenterological problems and Quincke's oedema), or general (*e*.*g*., anaphylactic shock). Some pathologies are sometimes abusively defined as allergies, although they do not depend on the above-recalled mechanism. This is the case, for example, of delayed-type hypersensitivity reactions. When IgG-induced mast cell activation is inhibited by a composition obtained according to the present invention, this composition can advantageously be used for preventing, alleviating or treating an autoimmune disease, such as for example rheumatoid arthritis, encephalomyelitis, multiple sclerosis, bullous pemphigoid, acute disseminated encephalomyelitis (ADEM), ankylosing spondylitis, antiphospholipid antibody syndrome (APS), autoimmune hepatitis, autoimmune oophoritis, celiac disease, Crohn's disease, gestational pemphigoid, Goodpasture's syndrome, Graves' disease, Guillain-Barré syndrome (GBS, also called acute inflammatory demyelinating polyneuropathy, acute idiopathic polyradiculoneuritis, acute idiopathic polyneuritis and Landry's ascending paralysis), Hashimoto's disease, idiopathic thrombocytopenic purpura, Kawasaki's disease, lupus erythematosus, myasthenia gravis, opsoclonus myoclonus syndrome (OMS), optic neuritis, Ord's thyroiditis, pemphigus, Reiter's syndrome, Sjögren's syndrome, Takayasu's arteritis, temporal arteritis (also known as "giant cell arteritis") and Wegener's granulomatosis. Any other auto-immune disease which depends on mast cell activation by antibodies can also be prevented or treated by a composition obtained according to the invention.

Compositions prepared according to the present invention can also be used for preventing, alleviating and/or treating type 2 diabetes, since LPS derived from the gut flora was recently reported to generate a chronic inflammation which favors the outcome of type 2 diabetes (Cani et al., 2007).

In a preferred embodiment, the *L. casei* strain used according to the present invention is a *L*. *casei ssp*. *paracasei*, for example the strain deposited at the CNCM (*Collection Nationale de Culture de Microorganismes,* 25 rue du Docteur Roux, Paris) under the number I-1518 on December 30, 1994.

In another embodiment, the *Bifidobacterium breve* strain used according to the present invention is the strain deposited at the CNCM under the number I-2219, on May 31, 1999.

According to a particular embodiment of the present invention, the composition prepared with a *L*. *casei* strain is a food supplement and/or a functional food. In the present text, a "food supplement" designates a product made from compounds usually used in foodstuffs, but which is in the form of tablets, powder, capsules, potion or any other form usually not associated with aliments, and which has beneficial effects for one's health. A "functional food" is an aliment which also has beneficial effects for one's health. In particular, food supplements and functional food can have a physiological effect - protective or curative - against a disease, for example against a chronic disease.

A particular composition prepared according to the present invention is a fermented dairy product.

Compositions obtained according to the present invention can also comprise at least one other bacterial strain selected from the genera *Lactobacillus, Lactococcus* and *Streptococcus,* for example at least one bacterial strain selected in the group consisting of *Streptococcus thermophilus* and *Lactobacillus bulgaricus*..

According to another embodiment, the composition prepared according to the present invention is a medicinal product.

In accordance with the invention, the bacteria, especially the *L. casei* bacteria, can be used in the form of whole bacteria which may be living or not. For example, whole irradiated *L*. *casei* can be used. Alternatively, bacteria can be used in the form of a bacterial lysate or in the form of bacterial fractions; the bacterial fractions suitable for this use can be chosen, for example, by testing their properties of inhibiting IgE-induced mast cell activation, for example by performing one of the assays disclosed in the experimental part below. Bacterial fractions are especially preferred, for example, in formulations targeting the mucous membrane of nose and sinus, the lung, In a preferred embodiment, the compositions obtained according to the present invention are formulated to enable a direct contact between mast cells and bacteria, bacterial lysate and/or the bacterial fraction (possibly partially degraded). The effects of *L. casei* and, to a lesser extent, of *B*. *breve* on the inhibition of mast cell activation, might also be observed with other bacterial strains. Libraries of bacterial strains, especially of already known probiotics, should be screened for identifying further strains able to inhibit mast cell activation.

The present invention hence also pertains to screening processes for identifying bacterial strains which can be used for preparing compositions for inhibiting mast cell activation, particularly activation by antibodies, especially for preventing, alleviating or treating a disease selected amongst allergies, autoimmune diseases and type 2 diabetes.

According to a first variant of the processes (first process) according to the invention, said process comprises the following steps:
(a) incubating mast cells with the bacteria to be screened, during at least one hour;
(b) optionally, removing said bacteria ;
(c) adding an activating agent to mast cells ; and
(d) measuring the activation of mast cells.

If necessary, washing steps are performed between steps b) and c), and between steps c) and d).

In the above process, the activating agent used in step (c) can be, for example, PMA, a calcium ionophore such as ionomycin, LPS, thapsigargine, preformed IgG/antigen complexes, or mixtures of two or more of those.

According to a second variant of the processes (second process) according to the invention, said process comprises the following steps:
(a) incubating mast cells with the bacteria to be screened, during at least one hour;
(b) optionally, removing said bacteria;
(c) incubating mast cells with IgE antibodies;
(d) adding specific antigen to mast cells ; and
(e) measuring the activation of mast cells.

Here again, washing steps can be performed, if necessary, between steps of the above process.

The activation of mast cells can be measured, for example, by one of the techniques described in the experimental part below, *i*.*e*., by measuring the level of beta-hexosaminidase and/or TNF-alpha released by the mast cells. Of course, the skilled artisan can use any other marker of mast cell activation, such as those described by Galli et al. (Nature immunol. 2005).

Depending on the nature and quantity of activating agent used and also on the technology used in the measuring step (d) or (e), the mast cells are incubated with said activating agent (in step (c) of the first process), or with the specific antigen (in step (d) of the second process) during a few minutes (*e*.*g*., from 5 to 30 minutes) or during a longer time (up to several hours). For example, the measurement of a compound present in mast cells granules, such as beta-hexosaminidase, will only require a few minutes' incubation, whereas if a cytokine is measured, such as TNF-alpha, the incubation in step c) must last at least one hour (from 1 to 5 hours).

Any other product released or secreted by mast cells and/or any cell alteration associated with mast cell activation may be also be measured.

The figures and experimental part below will further illustrate the present invention, without limiting its scope.

### FIGURE LEGENDS

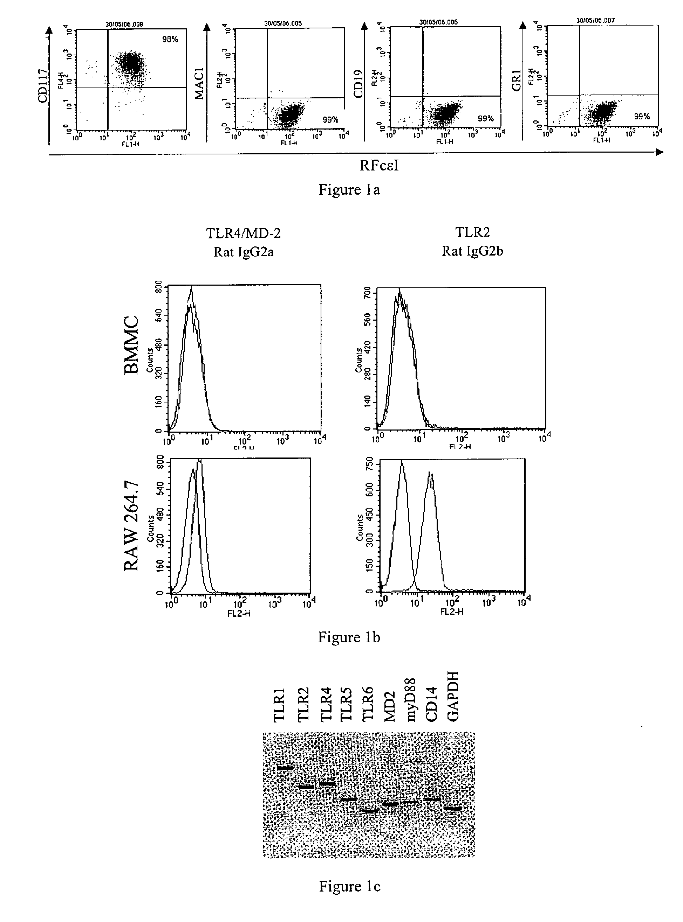

### Figure 1a: Phenotype of Bone Marrow derived Mast Cells (BMMC).

BMMC were preincubated for 10 minutes at 0°C with 10µg/ml 2.4G2 to prevent non specific binding of antibodies. They were then incubated for 30 minutes at 0°C with 10µg/ml FITC anti mouse FcεRI alpha, or 333ng/ml PE anti mouse CD19, or 133ng/ml PE anti mouse CD11b, or 100ng/ml PE anti mouse Ly-6G, or 200ng/ml APC anti mouse FcεRI alpha, and washed. Cell fluorescence was assessed by flow cytometry.

### Figure 1b: Expression of TLR proteins by BMMC.

BMMC were preincubated for 10 minutes at 0°C with 10µg/ml 2.4G2 to prevent non specific binding of antibodies. They were then incubated for 30 minutes at 0°C with 2µg/ml biotinylated monoclonal antibody to mouse TLR4 / MD2, or 2µg/ml biotinylated monoclonal antibody to mouse TLR2, and washed. They were subsequently incubated for 30 minutes at 0°C with 2µg/ml avidin, neutravidin, R-phycoerythrin conjugated, and washed. Cell fluorescence was assessed by flow cytometry.

### Figure 1c: Expression of TLR transcripts by BMMC.

RNA was extracted from BMMC using the RNeasy Mini Kit, and reverse transcribed using oligodT and the AMV reverse transcriptase. Resulting cDNA were analysed by PCR using primers specific for TLR1, TLR2, TLR4, TLR5, TLR6, MD2, MyD88, CD14 and GAPDH. Amplified fragments were subjected to electrophoresis in 2% agarose.

### Figure 2a: β-hexosaminidase release from BMMC exposed to probiotics.

BMMC were exposed for 20 minutes at 37°C to PBS, 10⁻⁷M PMA + 10⁻⁶M ionomycin or irradiated bacteria at a ratio of 1000 bacteria/cell. β-hexosaminidase release was assessed using an enzymatic colorimetric assay.

### Figure 2b: TNF-α secretion by BMMC exposed to probiotics.

BMMC were exposed for 3 hours at 37°C to PBS, 10⁻⁷M PMA + 10⁻⁶M ionomycin or irradiated bacteria at a ratio of 1000 bacteria/cell. TNF-α secretion was assessed using the L929 bioassay.

### Figure 3a: β-hexosaminidase release from BMMC exposed to probiotics prior to sensitization and challenge with Ag.

BMMC were preincubated overnight at 37°C with PBS or irradiated bacteria at a ratio of 1000 bacteria/cell. They were then sensitized for 1 hour at 37°C with 1µg/ml IgE anti-DNP and washed 3 times. They were subsequently challenged for 20 minutes at 37°C with 10ng/ml DNP-BSA. β-hexosaminidase release was assessed using an enzymatic colorimetric assay.

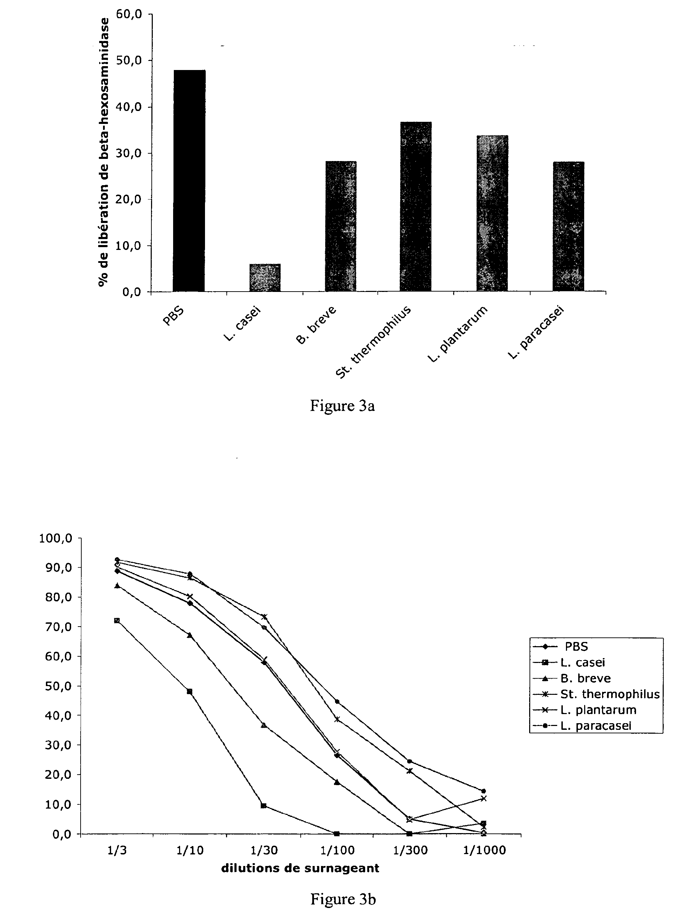

Figure 3b: TNF-α secretion by BMMC exposed to probiotics prior to sensitization and challenge with Ag.

BMMC were preincubated overnight at 37°C with PBS or irradiated bacteria at a ratio of 1000 bacteria/cell. They were then sensitized for 1 hour at 37°C with 1µg/ml IgE anti-DNP and washed 3 times. They were subsequently challenged for 3 hours at 37°C with 10ng/ml DNP-BSA. TNF-α secretion was assessed using the L929 bioassay.

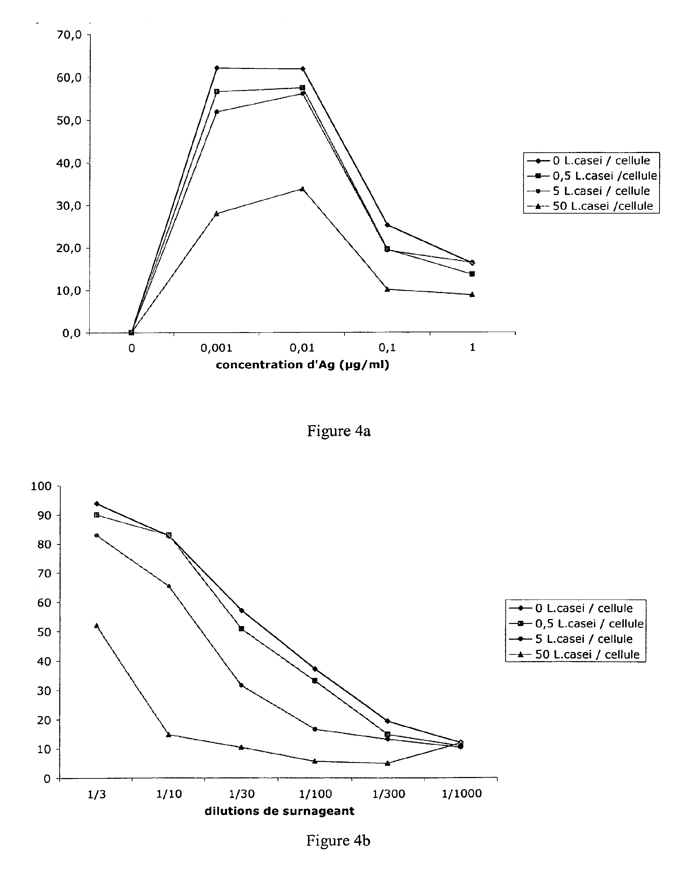

### Figure 4a: β-hexosaminidase release from BMMC exposed to live L. casei prior to sensitization and challenge with Ag.

BMMC were preincubated overnight at 37°C with PBS or alive bacteria at a ratio of 0.5, 5, 50 bacteria/cell, and washed 3 times. They were then sensitized for 1 hour at 37°C with 1µg/ml IgE anti-DNP and washed 3 times. They were subsequently challenged for 20 minutes at 37°C with the indicated concentration of DNP-BSA. β-hexosaminidase release was assessed using an enzymatic colorimetric assay.

### Figure 4b: TNF-α secretion by BMMC exposed to live L. casei prior to sensitization and challenge with Ag.

BMMC were preincubated overnight at 37°C with PBS or alive bacteria at a ratio of 0.5, 5, 50 bacteria/cell, and washed 3 times. They were then sensitized for 1 hour at 37°C with 1µg/ml IgE anti-DNP and washed 3 times. They were subsequently challenged for 3 hours at 37°C with 10ng/ml DNP-BSA. TNF-α secretion was assessed using the L929 bioassay.

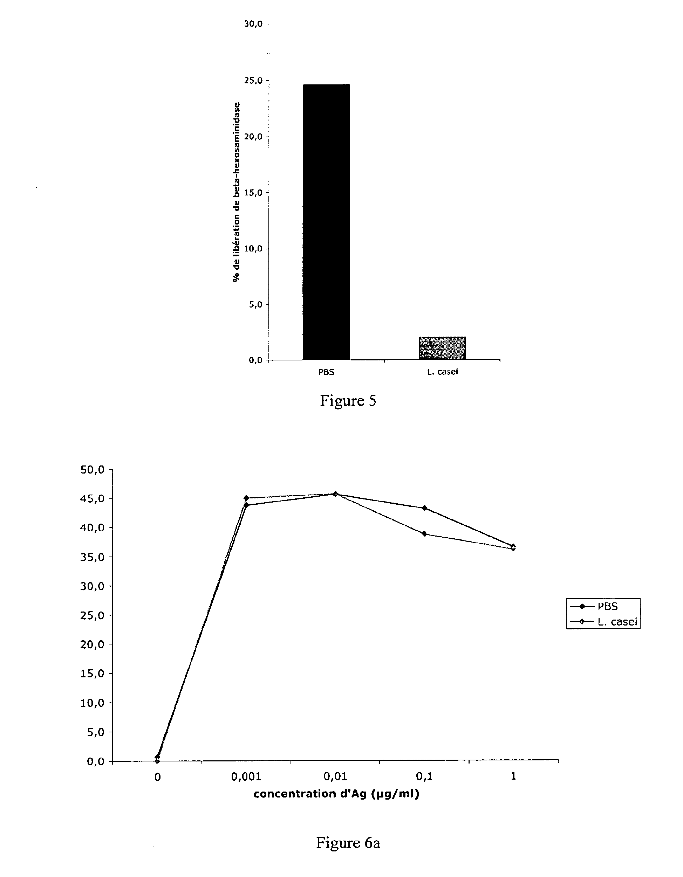

### Figure 5: β-hexosaminidase release from Peritoneal Cell-derived Mast Cells (PCMC) exposed to L. casei prior to sensitization and challenge with Ag.

PCMC were preincubated overnight at 37°C with PBS or irradiated bacteria at a ratio of 1000 bacteria/cell. They were then sensitized for 1 hour at 37°C with 1µg/ml IgE anti-DNP and washed 3 times. They were subsequently challenged for 20 minutes at 37°C with 10ng/ml DNP-BSA. β-hexosaminidase release was assessed using an enzymatic colorimetric assay.

### Figure 6a: β -hexosaminidase release from Rat Basophil Leukemia (RBL) exposed to L. casei prior to sensitization and challenge with Ag.

RBL cells were preincubated overnight at 37°C with PBS or irradiated bacteria at a ratio of 1000 bacteria/cell. They were then sensitized for 1 hour at 37°C with 1µg/ml IgE anti-DNP and washed 3 times. They were subsequently challenged for 20 minutes at 37°C with the indicated concentration of DNP-BSA. β -hexosaminidase release was assessed using an enzymatic colorimetric assay.

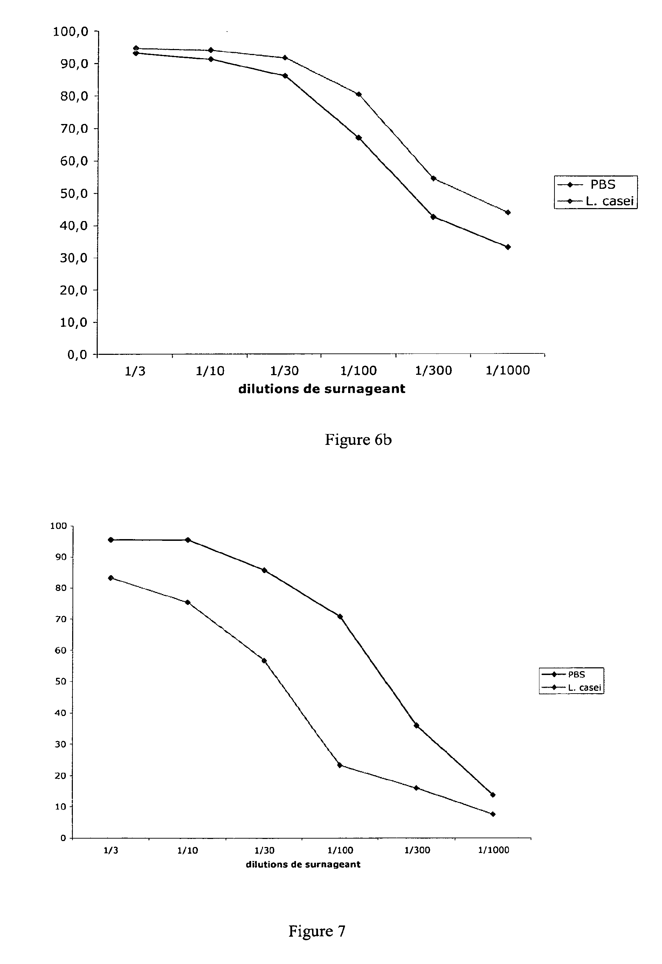

### Figure 6b: TNF-α secretion by RBL exposed to L. casei prior to sensitization and challenge with Ag.

RBL cells were preincubated overnight at 37°C with PBS or irradiated bacteria at a ratio of 1000 bacteria/cell. They were then sensitized for 1 hour at 37°C with 1µg/ml IgE anti-DNP and washed 3 times. They were subsequently challenged for 3 hours at 37°C with 10ng/ml DNP-BSA. TNF-α secretion was assessed using the L929 bioassay.

### Figure 7: TNF-α secretion by BMMC exposed to L. casei prior to challenge with PMA-ionomycin.

BMMC were preincubated overnight at 37°C with PBS or irradiated bacteria at a ratio of 1000 bacteria/cell, and washed 3 times. They were then challenged for 3 hours at 37°C with 10⁻⁷M PMA + 10⁻⁶M ionomycin. TNF-α secretion was assessed using the L929 bioassay.

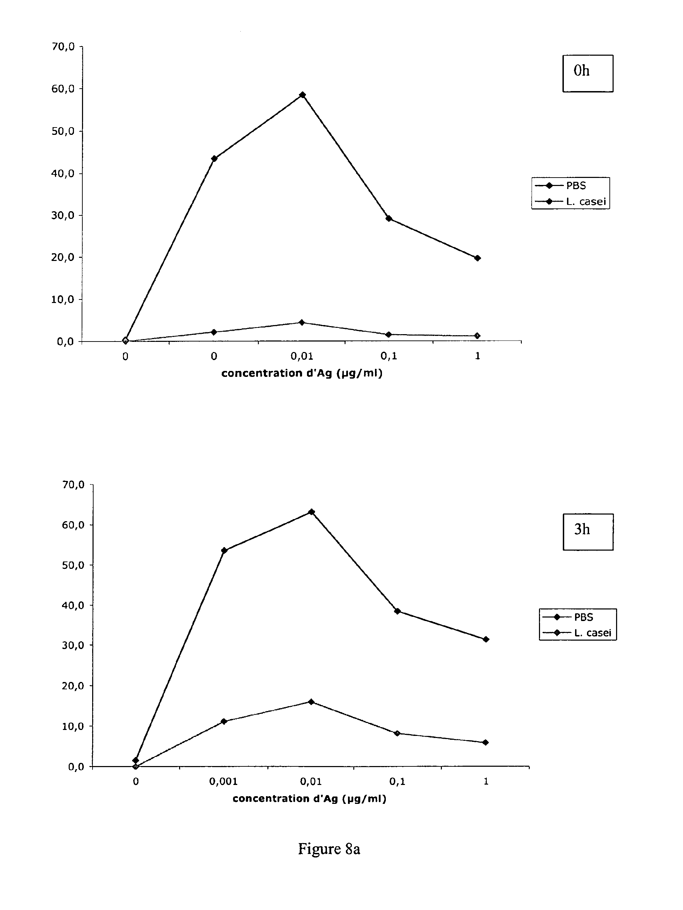

### Figure 8a: β-hexosaminidase release from BMMC exposed to L. casei and incubated for 3h in the absence of bacteria, prior to sensitization and challenge with Ag.

BMMC were preincubated overnight at 37°C with PBS or irradiated bacteria at a ratio of 1000 bacteria/cell, and washed 3 times. They were then incubated for 0 or 3 hours at 37°C in the absence of bacteria, sensitized for 1 hour at 37°C with 1µg/ml IgE anti-DNP, and washed 3 times. They were subsequently challenged for 20 minutes at 37°C with the indicated concentration of DNP-BSA. β-hexosaminidase release was assessed using an enzymatic colorimetric assay.

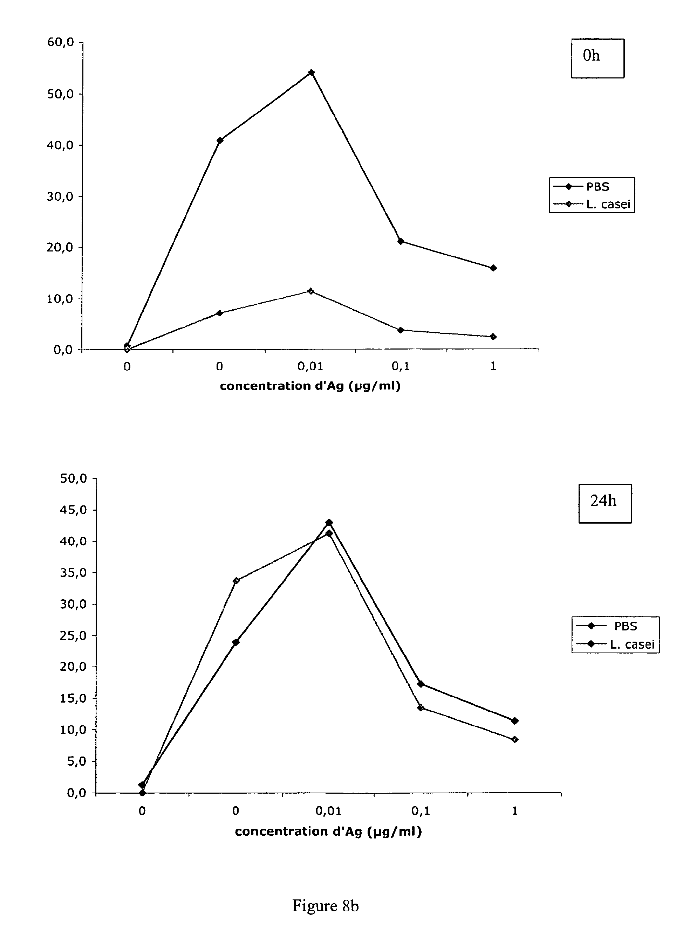

### Figure 8b: β-hexosaminidase release from BMMC exposed to L. casei and incubated for various periods of time in the absence of bacteria, prior to sensitization and challenge with Ag.

BMMC were preincubated overnight at 37°C with PBS or irradiated bacteria at a ratio of 1000 bacteria/cell, and washed 3 times. They were then incubated for 0 or 24 hours at 37°C in the absence of bacteria, sensitized for 1 hour at 37°C with 1µg/ml IgE anti-DNP, and washed 3 times. They were subsequently challenged for 20 minutes at 37°C with the indicated concentration of DNP-BSA. β-hexosaminidase release was assessed using an enzymatic colorimetric assay.

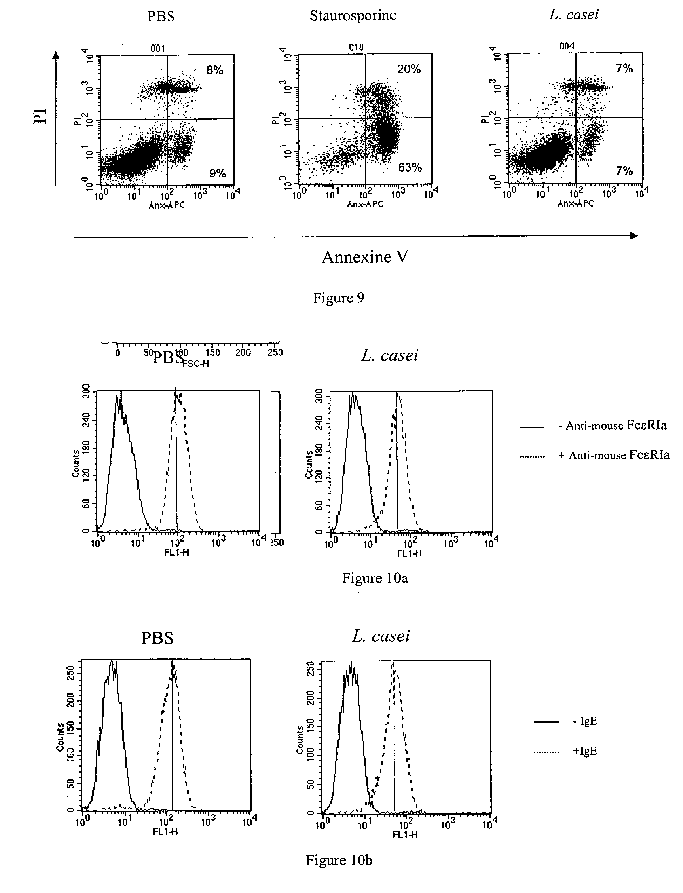

### Figure 9: Propidium Iodide (PI)/Annexin V labelling of BMMC exposed to L. casei.

BMMC were preincubated overnight at 37°C with PBS, 250ng/ml staurosporine or irradiated bacteria at a ratio of 1000 bacteria/cell, and washed 3 times. They were then incubated for 30 minutes at 0°C with 0.5 µg/ml PI and 0.5 µl Annexin V-APC (BD Biosciences). Cell fluorescence was assessed by flow cytometry.

### Figure 10a: FcεRI expression by BMMC exposed to L. casei.

BMMC were preincubated overnight at 37°C with PBS or irradiated bacteria at a ratio of 1000 bacteria/cell, and washed 3 times. They were then incubated for 10 minutes at 0°C with 10µg/ml 2.4G2 to prevent non specific binding of antibodies, incubated for 30 minutes at 0°C with 10µg/ml FITC anti mouse FcεRI alpha, and washed. Cell fluorescence was assessed by flow cytometry.

### Figure 10b: IgE binding on BMMC exposed to L. casei.

BMMC were preincubated overnight at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently incubated for 30 minutes at 0°C with 30µg/ml FITC-labeled Fab'2 goat anti-mouse, and washed. Cell fluorescence was assessed by flow cytometry.

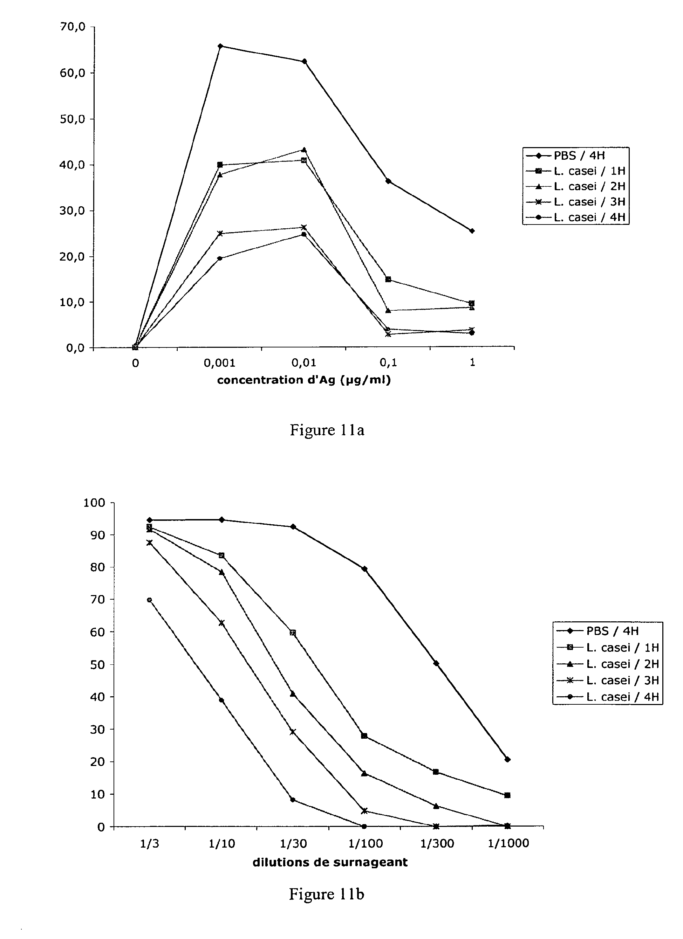

### Figure 11a: β-hexosaminidase release from BMMC exposed to L. casei for various periods of time, prior to sensitization and challenge with Ag.

BMMC were preincubated 4 hours at 37°C with PBS or 1, 2, 3, 4 hours at 37°C with irradiated bacteria at a ratio of 1000 bacteria/cell, and washed 3 times. They were then sensitized for 1 hour at 37°C with 1µg/ml IgE anti-DNP, and washed 3 times. They were subsequently challenged for 20 minutes at 37°C with the indicated concentration of DNP-BSA. β -hexosaminidase release was assessed using an enzymatic colorimetric assay.

### Figure 11b: TNF-α secretion by BMMC exposed to L. casei for various periods of time, prior to sensitization and challenge with Ag.

BMMC were preincubated 4 hours at 37°C with PBS or 1, 2, 3, 4 hours at 37°C with irradiated bacteria at a ratio of 1000 bacteria/cell, and washed 3 times. They were then sensitized for 1 hour at 37°C with 1µg/ml IgE anti-DNP, and washed 3 times. They were subsequently challenged for 3 hours at 37°C with 10ng/ml DNP-BSA. TNF-α secretion was assessed using the L929 bioassay.

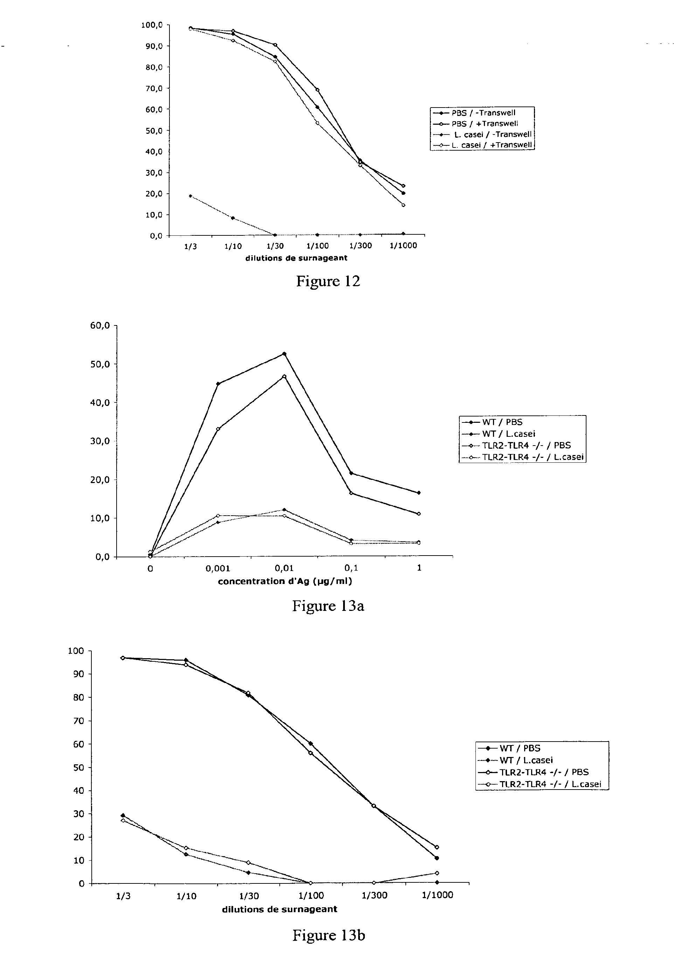

### Figure 12: TNF-α secretion by BMMC exposed to L. casei but separated by a membrane, prior to sensitization and challenge with Ag.

BMMC were preincubated overnight at 37°C with PBS or irradiated bacteria at a ratio of 1000 bacteria/cell in the presence or absence of a transwell, and washed 3 times. They were then sensitized for 1 hour at 37°C with 1µg/ml IgE anti-DNP, and washed 3 times. They were subsequently challenged for 3 hours at 37°C with 10ng/ml DNP-BSA. TNF-α secretion was assessed using the L929 bioassay.

### Fig 13a: β-hexosaminidase release from TLR-2/TLR-4-deficient BMMC exposed to L. casei prior to sensitization and challenge with Ag.

BMMC from wild type and TLR-2/TLR-4-deficient mice were preincubated overnight at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently challenged for 20 minutes at 37°C with the indicated concentration of DNP-BSA. β-hexosaminidase release was assessed using an enzymatic colorimetric assay.

### Fig 13b: TNF-α, secretion by TLR-2/TLR-4-deficient BMMC exposed to L. casei prior to sensitization and challenge with Ag.

BMMC from wild type and TLR-2/TLR-4-deficient mice were preincubated overnight at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently, challenged for 3 hours at 37°C with 10ng/ml DNP-BSA. TNF-α secretion was assessed using the L929 bioassay.

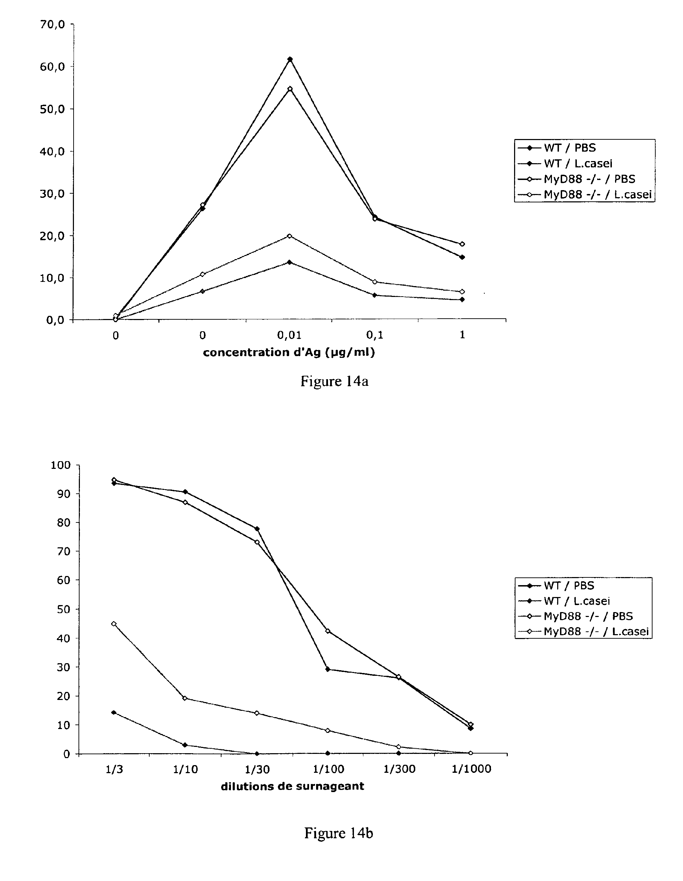

### Fig 14a: β-hexosaminidase release from MyD88-deficient BMMC exposed to L. casei prior to sensitization and challenge with Ag.

BMMC from wild type and MyD88-deficient mice were preincubated overnight at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently challenged for 20 minutes at 37°C with the indicated concentration of DNP-BSA. β-hexosaminidase release was assessed using an enzymatic colorimetric assay.

### Fig 14b: TNF-α secretion by MyD88-deficient BMMC exposed L. casei prior to sensitization and challenge with Ag.

BMMC from wild type and MyD88-deficient mice were preincubated overnight at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently challenged for 3 hours at 37°C with 10ng/ml DNP-BSA. TNF-α secretion was assessed using the L929 bioassay.

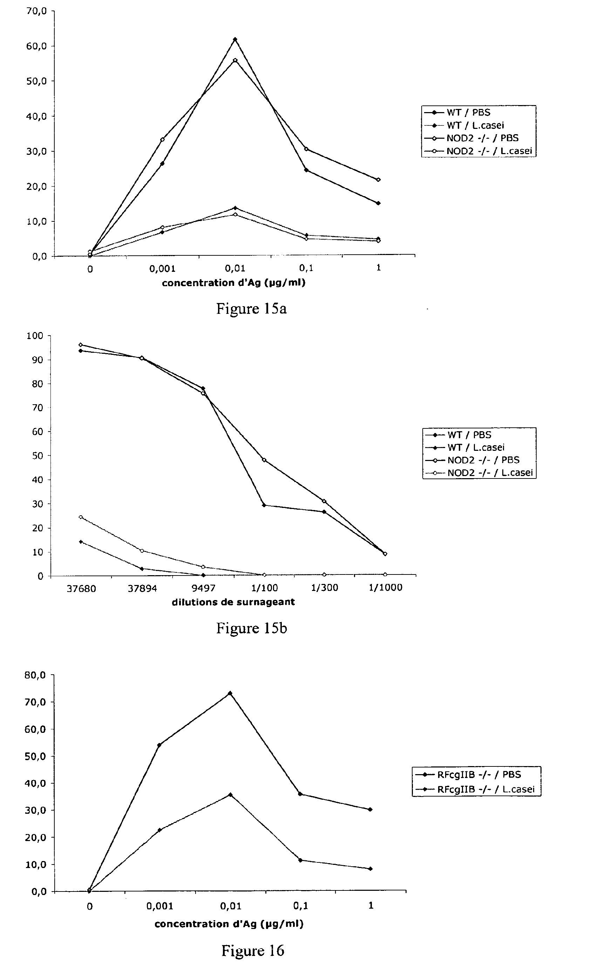

### Fig15a: β-hexosaminidase release from NOD2-deficient BMMC exposed to L. casei prior to sensitization and challenge with Ag.

BMMC from wild type and NOD2-deficient mice were preincubated overnight at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently challenged for 20 minutes at 37°C with the indicated concentration of DNP-BSA. β-hexosaminidase release was assessed using an enzymatic colorimetric assay.

### Fig15b: TNF-α secretion by NOD2-deficient BMMC exposed to L. casei prior to sensitization and challenge with Ag.

BMMC from wild type and NOD2-deficient mice were preincubated overnight at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently challenged for 3 hours with 10ng/ml DNP-BSA. TNF-α secretion was assessed using the L929 bioassay. **Fig16****:** β**-hexosaminidase release from Fc**γ**RIIB-deficient BMMC exposed to *L. casei* prior to sensitization and challenge with Ag.**

BMMC from wild type and FcγRIIB-deficient mice were preincubated 4 hours at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently challenged for 20 minutes at 37°C with the indicated concentration of DNP-BSA. β-hexosaminidase release was assessed using an enzymatic colorimetric assay.

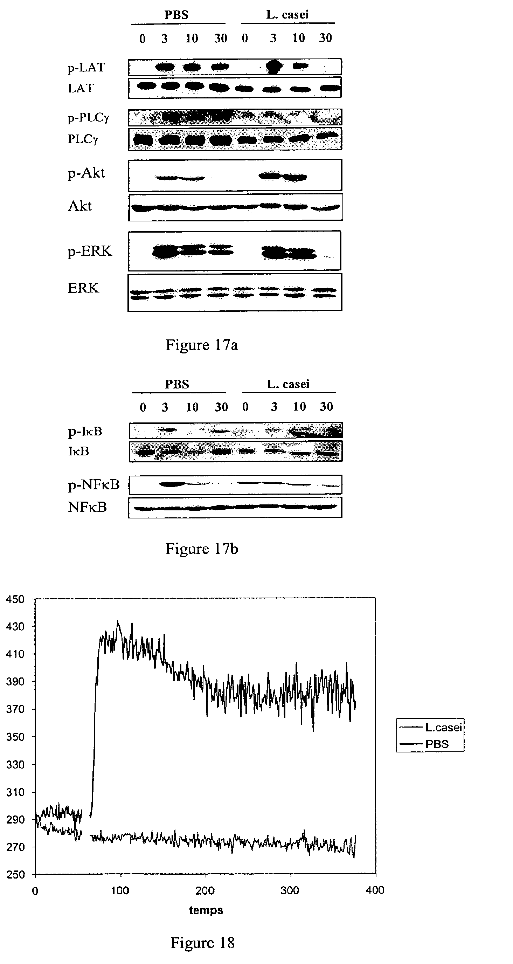

### Fig17a: Intracellular signaling proteins expression and phosphorylation in BMMC exposed to L. casei prior to sensitization and challenge with Ag.

BMMC were preincubated overnight at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently challenged for 0, 3, 10 or 30 minutes with 10ng/ml DNP-BSA and lysed. Cell lysates were analysed by SDS-PAGE followed by Western blot using anti-pLAT, anti-LAT, anti-pPLCγ1, anti-PLCγ1, anti-pERK, anti-ERK, anti-pAkt, anti-Akt.

### Fig17b: Transcription factor activation in BMMC exposed to L. casei prior to sensitization and challenge with Ag.

BMMC were preincubated overnight at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently challenged for 0, 3, 10 or 30 minutes with 10ng/ml DNP-BSA and lysed. Cell lysates were analysed by SDS-PAGE followed by Western blot using anti-pNFκB, anti-NFκB, anti-pIκBα, anti-IκBα.

### Fig18: Calcium influx in BMMC exposed to L. casei prior to sensitization and challenge with Ag.

BMMC were preincubated overnight at 37°C with PBS or irradiated bacteria at a ratio of 1000 bacteria/cell, and washed 3 times. They were then sensitized for 1 hour at 37°C with 1µg/ml IgE anti-DNP, and washed 3 times. They were subsequently loaded for 30 minutes at room temperature with 5µM of the calcium indicator dye Fluo3AM, washed 3 times, and challenged at 37°C with 10ng/ml DNP-BSA. Variation of cell fluorescence upon cell stimulation was assessed by flow cytometry.

### EXAMPLES

The experiments described below have been performed using the materials and methods which follow:

### Cells culture

Femoral bone marrow cells were collected and cultured in Opti-MEM + GlutaMAX-I supplemented with 10% FCS, 0,2% 2-mercaptoethanol, 100IU/ml penicillin, 100 µg/ml streptomycin (complete Opti-MEM), and 4% supernatant of X63 transfectants secreting murine IL-3 (Dr. P. Dubreuil, Institut de Cancérologie et d'Immunologie, Marseille, France). Cultures were passaged every 3 days by resuspending the pelleted cells in fresh culture medium at a concentration of 3 X 10⁵/ml.

Peritoneal cells were collected from mice injected with 2 ml of RPMI 1640 i.p. They were seeded at 1 X 10⁶/ml in complete Opti-MEM supplemented with 4% supernatant of CHO transfectants secreting murine SCF (Dr. P. Dubreuil). Twenty-four hours later, nonadherent cells were removed and fresh culture medium was added to adherent cells. Three days later, nonadherent cells and adherent cells recovered with trypsin-SDTA were harvested, pelleted, and resuspended in fresh culture medium at a concentration of 3 X 10⁵/ml The same procedure was repeated twice a week. 3-9 wk old cultures were used for experiments. Culture reagents were obtained from Invitrogen Life Technologies.

Rat Basophils Leukemia Cells were cultured in RPMI 1640 + GlutaMAX-I supplemented with 10% FCS, 100IU/ml penicillin and 100 µg/ml streptomycin.

Raw 264.7 cells were cultured in DMEM + GlutaMAX-I supplemented with 10% FCS, 100IU/ml penicillin and 100 µg/ml streptomycin.

### Cell preincubation

Cells were preincubated with bacteria, PBS or staurosporine in Opti-MEM + GlutaMAX-I supplemented with 10% FCS and 4% supernatant of X63 transfectants secreting murine IL-3.

### Cell sensitization

Cells were sensitized with IgE anti-DNP in RPMI 1640 + GlutaMAX-I supplemented with 10% FCS and 4% Hepes.

### Cell stimulation

Cells were stimulated with bacteria, PMA/ionomycin or DNP-BSA in RPMI 1640 + GlutaMAX-I supplemented with 10% FCS and 4% Hepes.

### Cell labelling

Cells were incubated with 2.4G2 and biotin, PE, FITC, APC-labelled antibodies in PBS supplemented with 0.2% FCS.

Cells were incubated with PI/Annexin V-APC in binding buffer (BD Biosciences).

Cells were incubated with Fluo3AM in RPMI 1640 + GlutaMAX-I supplemented with 0.2% pluronic.

### Example 1: inhibition of IgE-induced activation of BMMC by Lactobacillus casei

The model cells used are murine Bone Marrow-derived Mast Cells (BMMC), in which the expression of FcεRI, FcγRIIIA and FcγRIIB was checked. They also express the Stem Cell Factor receptor kit (CD117), but no macrophage (Macl), B cell (CD19), or granulocytes (GR1) markers (Fig. 1a). BMMC did not detectably express membrane TLR-2 or TLR-4 as assessed by indirect immunofluorescence with available antibodies (Fig. 1b), but contained transcripts encoding TLR-1, 2, 4, 5, 6, MD-2, MyD88 and CD14, as assessed by RT-PCR analysis (Fig. 1c). When sensitized with IgE antibodies and challenged with specific antigen, BMMC release β-hexosaminidase and secrete TNF-α.

Five bacterial strains were tested for their effects on mast cell activation:
- *Lactobacillus casei ssp*. *paracasei*, which was deposited at the CNCM (*Collection Nationale de Culture de Microorganismes,* 25 rue du Docteur Roux, Paris) under the number I-1518, on December 30, 1994, designated as "L. *casei"* in the examples described herein;
- *Lactobacillus paracasei* DN-114 120;
- *Bifidobacterium breve* deposited at the CNCM under the number I-2219, on May 31, 1999;
- *Lactobacillus plantarum* DSM 9843;
- and *Streptococcus thermophilus* deposited at the CNCM under the number I-2273,, on January 24, 2002.

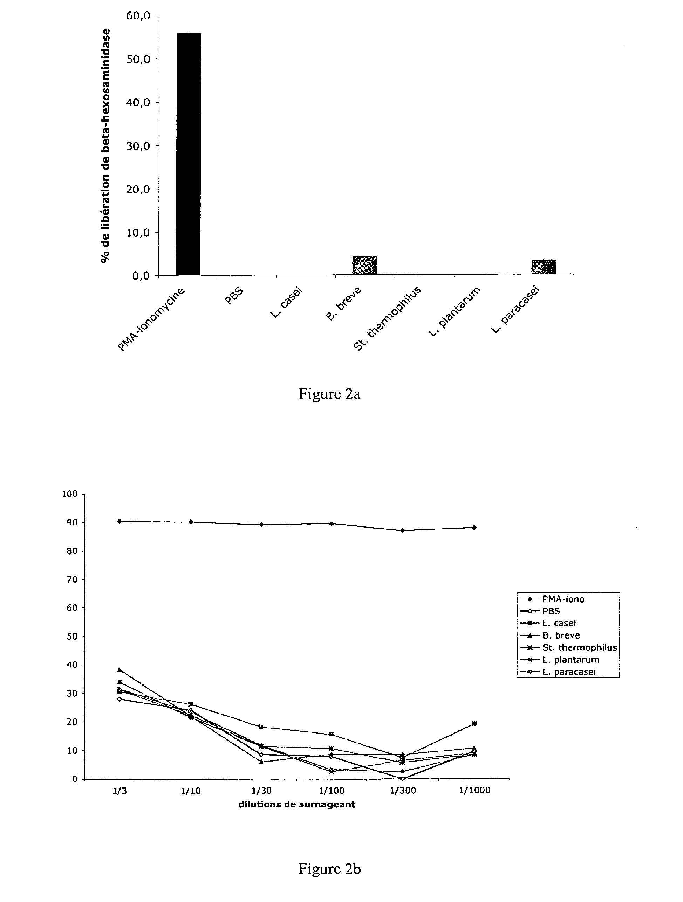

These bacteria were first tested for their ability to activate mast cells. To that aim, the bacteria were irradiated, resuspended in PBS and incubated with BBMC (1000 bacteria/cell). Among the 6 bacterial strains tested, none induced BMMC to release β-hexosaminidase or to secrete TNF-α, when incubated for 20 min or 3hr, respectively (Fig. 2a and 2b).

The inventors then investigated whether a previous exposure of BMMC to bacteria would affect the subsequent IgE-induced biological responses of mast cells. They found that when incubated overnight with BMMC (at a ratio of 1000 irradiated bacteria/cell), several strains inhibited both the release of β-hexosaminidase and the secretion of TNF-α. Others did not. One strain, *L. casei,* was markedly more efficient than other bacteria (Fig. 3a and 3b). Under the same conditions, live *L. casei* also inhibited mast cell activation, and comparable inhibitions were induced at a lower bacteria/cell ratio (Fig. 4a and 4b). For practical reasons, irradiated bacteria were used in subsequent experiments. The results obtained are described below.

### A. General features of L. casei-induced inhibition

*1. L. casei* equally affected IgE-induced responses in BMMC and in a novel model of primary serosal-type mature mast cells that was developed in the laboratory (Malbec et al., 2007) (Fig. 5), but not the responses of the tumor mast cell line RBL-2H3 (Fig. 6a and 6b);
2. *L. casei* inhibited not only IgE-, but also PMA+ionomycin-induced responses of BMMC (Fig. 7), which demonstrates that *L. casei* inhibits a non-specific activation which bypasses membrane antibody receptors and the early steps of intracellular signalling;
3. inhibition was transient (Fig. 8a and 8b);
4. *L. casei* did not decrease the viability of mast cells (Fig. 9);
5. *L. casei* decreased slightly the expression of FcεRI by BMMC, the sensitization of which by IgE antibodies was not affected.

### B. Experimental conditions for inhibition to occur

1. The inhibition of mast cell responses by previous incubation with *L. casei* is time-dependent (Fig. 11a and 11b);
2. inhibition could not be accounted for by soluble products present in *L. casei* supernantant and it was not induced by lactic acid;
3. supporting this observation, inhibition was prevented if *L. casei* and BMMC were separated by a 0.4-µm transwell membrane during incubation, indicating that it requires a direct contact between cells and bacteria (Fig. 12).

### C. Mechanisms involved in inhibition of mast cell activation by L. casei

*1. L. casei* inhibited IgE-induced responses of BMMC derived from a) TLR-2/TLR-4-deficient mice (Fig. 13a and 13b), b) MyD88-deficient mice (Fig. 14a and 14b), c) NOD2-deficient mice (Fig. 15a and 15b), and d) FcγRIIB-deficient mice (Fig. 16), as efficiently as responses of BMMC from wt mice;
*2. L. casei* decreased the expression of several signaling molecules as assessed by Western blotting (Fig. 17);
*3. L. casei* inhibited both early and late FcεRI-dependent phosphorylation events (Fig. 17);
*4. L. casei* enhanced NF-κB phosphorylation but decreased IκB degradation which is required for the nuclear translocation of NFκB (Fig. 17); and
5. *L. casei* profoundly inhibited both IgE-induced and ionomycin-induced increase of the intracellular concentration of Ca²⁺ (Fig. 18).
These results indicate that a few-hour exposure of mast cells to *L. casei* affected not only FcεRI signalling, leading to the activation of transcription factors and to Ca²⁺ responses, but also activation signals that by-pass FcεRI (PMA+ionomycicn). As a consequence, IgE-induced mast cells secretory responses (both degranulation and cytokine secretion) were markedly inhibited.

### Example 2: Inhibition of IgE-induced in vivo responses by L. casei

The *in vivo* effects of *L. casei* are primarily investigated in mice exposed to *L. casei* by gavage. *L*. *casei* is first studied as for its ability to inhibit passive local or systemic anaphylaxis. *L. casei* is then studied as for its ability to inhibit antigen-induced release of mast cell mediators by IgE-sensitized ileum segments from mice submitted to *L. casei* gavage. Finally, the consequences of *L. casei* gavage are explored in murine models of allergies (allergic asthma and food allergy), as well as in models of autoimmune inflammatory diseases (rheumatoid arthritis, encephalomyelitis) established in the laboratory.

### Example 3: Effects of L. casei on human mast cells and basophils in vitro

Blood basophils or cord blood-derived mast cells (CBMC) are exposed to *L. casei* under similar conditions as mouse mast cells and their responses to a stimulation via IgE is examined.

### Example 4: Effects of L. casei on allergy and autoimmunity in humans

Groups of normal and allergic patients, fed with Actimel® or placebo, are included in a clinical trial. Patients with autoimmune diseases (rheumatoid arthritis, multiple sclerosis or bullous pemphigoid) are also included, in a separate group, as well as patients with type 2 diabetes.

### Example 5: Search for new probiotics of interest

An automated *in vitro* assay in multi-well plates is established in order to screen a large library of probiotics. If this screening discloses new probiotics of potential interest, these are examined using the same experimental procedures as for *L. casei.*

### REFERENCES

Arock, M., Ross, E., Lai-Kuen, R., Averlant, G., Gao, Z., and Abraham, S. N. (1998). Phagocytic and tumor necrosis factor alpha response of human mast cells following exposure to gram-negative and gram-positive bacteria. Infect Immun 66, 6030-6034.

Benhamou, M., Bonnerot, C., Fridman, W. H., and Daeron, M. (1990). Molecular heterogeneity of murine mast cell Fc gamma receptors. J Immunol 144, 3071-3077.

Cani, P. D., Amar, J., Iglesias, M. A., Poggi, M., Knauf, C., Bastelica, D., Neyrinck, A. M., Fava, F., Tuohy, K. M., Chabo, C., et al. (2007). Metabolic endotoxemia initiates obesity and insulin resistance. Diabetes 56, 1761-1772.

Daeron, M., Bonnerot, C., Latour, S., and Fridman, W. H. (1992). Murine recombinant Fc gamma RIII, but not Fc gamma RII, trigger serotonin release in rat basophilic leukemia cells. J Immunol 149, 1365-1373.

Daeron, M., Latour, S., Malbec, O., Espinosa, E., Pina, P., Pasmans, S., and Fridman, W. H. (1995a). The same tyrosine-based inhibition motif, in the intracytoplasmic domain of Fc gamma RIIB, regulates negatively BCR-, TCR-, and FcR-dependent cell activation. Immunity 3, 635-646.

Daeron, M., Malbec, O., Latour, S., Arock, M., and Fridman, W. H. (1995b). Regulation of high-affinity IgE receptor-mediated mast cell activation by murine low-affinity IgG receptors. J Clin Invest 95, 577-585.

Daeron, M., Prouvost-Danon, A., and Voisin, G. A. (1980). Mast cell membrane antigens and Fc receptors in anaphylaxis. II. Functionally distinct receptors for IgG and for IgE on mouse mast cells. Cell Immunol 49, 178-189.

Galli, S. J., Gordon, J. R., and Wershil, B. K. (1991). Cytokine production by mast cells and basophils. Curr Opin Immunol 3, 865-872.

Galli, S. J., Nakae, S., and Tsai, M. (2005). Mast cells in the development of adaptive immune responses. Nat Immunol 6, 135-142.

Hazenbos, W. L., Gessner, J. E., Hofhuis, F. M., Kuipers, H., Meyer, D., Heijnen, I. A., Schmidt, R. E., Sandor, M., Capel, P. J., Daeron, M., et al. (1996). Impaired IgG-dependent anaphylaxis and Arthus reaction in Fc gamma RIII (CD16) deficient mice. Immunity 5, 181-188.

Kaplan, A. P. (2001). Chemokines, chemokine receptors and allergy. Int Arch Allergy Immunol 124, 423-431.

Latour, S., Bonnerot, C., Fridman, W. H., and Daeron, M. (1992). Induction of tumor necrosis factor-alpha production by mast cells via Fc gamma R. Role of the Fc gamma RIII gamma subunit. J Immunol 149, 2155-2162.

Lee, D. M., Friend, D. S., Gurish, M. F., Benoist, C., Mathis, D., and Brenner, M. B. (2002). Mast cells: a cellular link between autoantibodies and inflammatory arthritis. Science 297, 1689-1692.

Malbec, O., Roget, K., Schiffer, C., Iannascoli, B., Dumas, A. R., Arock, M., and Daeron, M. (2007). Peritoneal cell-derived mast cells: an in vitro model of mature serosal-type mouse mast cells. J Immunol 178, 6465-6475.

Miller, H. R., and Pemberton, A. D. (2002). Tissue-specific expression of mast cell granule serine proteinases and their role in inflammation in the lung and gut. Immunology 105, 375-390.

Nigrovic, P. A., Binstadt, B. A., Monach, P. A., Johnsen, A., Gurish, M., Iwakura, Y., Benoist, C., Mathis, D., and Lee, D. M. (2007). Mast cells contribute to initiation of autoantibody-mediated arthritis via IL-1. Proc Natl Acad Sci U S A 104, 2325-2330.

Robbie-Ryan, M., Tanzola, M. B., Secor, V. H., and Brown, M. A. (2003). Cutting edge: both activating and inhibitory Fc receptors expressed on mast cells regulate experimental allergic encephalomyelitis disease severity. J Immunol 170, 1630-1634.

Shelley, O., Murphy, T., Lederer, J. A., Mannick, J. A., and Rodrick, M. L. (2003). Mast cells and resistance to peritoneal sepsis after burn injury. Shock 19, 513-518.

Supajatura, V., Ushio, H., Nakao, A., Okumura, K., Ra, C., and Ogawa, H. (2001). Protective roles of mast cells against enterobacterial infection are mediated by Toll-like receptor 4. J Immunol 167, 2250-2256.

Triggiani, M., Oriente, A., de Crescenzo, G., and Marone, G. (1995). Metabolism of lipid mediators in human basophils and mast cells. Chem Immunol 61, 135-147.

## Claims

1. Use of a *L*. *casei* strain and/or a B. *breve* strain, for the preparation of a composition for inhibiting mast cell activation.

2. The use of claim 1, for the preparation of a composition for preventing IgE-induced mast cell activation.

3. The use of claim 2, for the preparation of a composition for preventing, alleviating or treating an allergy.

4. The use of claim 1, for the preparation of a composition for preventing IgG-induced mast cell activation.

5. The use of claim 4, for the preparation of a composition for preventing, alleviating or treating an autoimmune disease.

6. The use of claim 1, for the preparation of a composition for preventing, alleviating or treating type 2 diabetes.

7. The use of any of claims 1 to 6, wherein said *L. casei* strain is a *L*. *casei ssp*. *Paracasei* strain.

8. The use of any of claims 1 to 7, wherein said *L*. *casei* strain is the strain deposited at the CNCM on December 30, 1994, under the number I-1518.

9. The use of any of claims 1 to 8, wherein said *Bifidobacterium breve* strain is the strain deposited at the CNCM under the number I-2219, on May 31, 1999.

10. The use of any of claims 1 to 9, wherein said composition is a food supplement and/or a functional food.

11. The use of any of claims 1 to 10, wherein said composition is a fermented dairy product.

12. The use of any of claims 1 to 11, wherein said composition further comprises at least one other bacterial strain selected from the genera *Lactobacillus, Lactococcus* and *Streptococcus.*

13. The use of claim 12, wherein said composition comprises at least one bacterial strain selected in the group consisting of *Streptococcus thermophilus* and *Lactobacillus bulgaricus.*

14. The use of any of claims 1 to 9, 12 and 13, wherein said composition is a medicinal product.

15. The use according to any of claims 1 to 14, wherein said bacteria are used alive.

16. The use according to any of claims 1 to 15, wherein said bacteria are used as whole dead cells.

17. The use according to any of claims 1 to 15, wherein said composition comprises a bacterial lysate or a bacterial fraction of said bacteria.

18. The use according to any of claims 1 to 17, wherein said composition is formulated so that uptake of this composition leads to a direct contact between mast cells and the bacteria, bacterial lysate and/or bacterial fraction comprised in said composition.

19. A screening process for identifying bacterial strains which can be used for preparing compositions for inhibiting mast cell activation by antibodies, wherein said process comprises the following steps:
(a) incubating mast cells with the bacteria to be screened, during at least one hour;
(b) optionally, removing said bacteria ;
(c) adding an activating agent to mast cells ; and
(d) measuring the activation of mast cells.

20. The screening process of claim 19, wherein the activating agent used in step (c) is selected in the group consisting of preformed IgG/antigen complexes, calcium ionophore, LPS, PMA, ionomycin, thapsigargine and mixtures of two or more of those.

21. A screening process for identifying bacterial strains which can be used for preparing compositions for inhibiting mast cell activation by antibodies, wherein said process comprises the following steps:
(a) incubating mast cells with the bacteria to be screened, during at least one hour;
(b) optionally, removing said bacteria;
(c) incubating mast cells with IgE antibodies;
(d) adding specific antigen to mast cells ; and
(e) measuring the activation of mast cells.

22. The screening process according to any of claims 19 to 21, wherein step (d) or (e) is performed by measuring the level of beta-hexosaminidase and/or TNF-alpha released by the mast cells and/or any product released or secreted by mast cells and/or any cells alteration associated with mast cell activation.
